# EUROPEAN PATENT SPECIFICATION

(11) **EP 1 872 757 B1**
(45) Date of publication and mention of the grant of the patent: **16.11.2011**
(21) Application number: 07252592.6
(22) Date of filing: 26.06.2007
(51) Int. Cl.: A61F 13/02, B32B 5/26

(54) **Medical adhesive tape or sheet**
Medizinisches Klebeband oder Klebefolie
Bande ou feuille adhésive à usage médical

(30) Priority: 30.06.2006 JP 2006182581
(43) Date of publication of application: 02.01.2008
(73) Proprietor: Nitto Denko Corporation, Ibaraki-shi, Osaka 567-8680 (JP)
(72) Inventor: Hatanaka, Hiroshi c/o Nitto Denko Corporation, Osaka 567-8680 (JP); Ueda, Yuko c/o Nitto Denko Corporation, Osaka 567-8680 (JP); Funakoshi, Yoshio c/o Nitto Denko Corporation, Osaka 567-8680 (JP); Kasahara, Tsuyoshi c/o Nitto Denko Corporation, Osaka 567-8680 (JP); Furumori, Kenji c/o Nitto Denko Corporation, Osaka 567-8680 (JP)
(74) Representative: Price, Nigel John King

(56) References cited:
- US-A- 3 085 024
- US-A- 4 041 203
- US-A1- 2003 040 691

## Description

The present invention relates to a medical adhesive tape or sheet, and more particularly relates to a medical adhesive tape or sheet useful for external application in the medical hygiene field.

Medical adhesive tape or sheet has, for example, a structure wherein an adhesive layer is laminated on one surface of a support, and is used by application of the adhesive layer to the skin surface to be applied to. As such a medical adhesive tape, surgical tapes and the like are known. However, since they use non-woven fabric such as pulp and the like as a support, they offer good texture, but are difficult to cut easily with hands. Thus, cutting with scissors and the like is needed, problematically degrading the workability.

Regarding the hand-tearability of an adhesive tape, for example, JP-A-09-502111 proposes an adhesive tape having a given perforated line, which contains a single layer support made of a binder containing non-woven web. By this method, an adhesive tape having good hand-tearability can be obtained. Even when the tape could be cut with hands, the section tends to be non-uniform, which is far from superior.

When a non-woven fabric is used as a support, an adhesive directly applied to form an adhesive layer penetrates the support due to many voids in the non-woven fabric, and the adhesive leaks from the backside of the support, thus causing penetration. Therefore, when a non-woven fabric is used as a support, an adhesive is not directly applied to the support. Instead, a method comprising applying an adhesive to a sheet such as release paper and the like to form an adhesive layer in advance, and adhering the sheet with an adhesive layer laminated thereon to a support to allow transfer of the adhesive layer has been employed. However, this method is disadvantageous in terms of production cost, since redundant sheet is mostly discarded.

As a method for preventing penetration of an adhesive, a method comprising treating a support with a sealer is known. When treated with a sealer, however, the support comes to have a low void ratio to easily cause stuffiness. Due to degraded texture, moreover, the end portion of an adhesive tape may stimulate the skin to cause skin irritation and the like. Moreover, since a comparatively large amount of a sealer is needed, production of an economical adhesive tape becomes difficult.

The present invention has been made in view of such situation and aims at providing a medical adhesive tape or sheet having a good cross-section, permitting easy cutting with hands, and capable of having a superior texture.

The present inventors have conducted intensive studies in an attempt to solve the aforementioned problems and found that the above-mentioned object can be achieved by laminating multiple layers of non-woven fabrics having different fiber diameters, seal-treating the laminate to give a support, and perforating at least the support at a particular ratio, which resulted in the completion of the present invention.

Accordingly, the present invention provides the following. A medical adhesive tape or sheet comprising a support, which is a multi-layer laminate of non-woven fabrics having at least a first non-woven fabric layer made of an ultrafine fiber and a second non-woven fabric layer made of a fiber having a fiber diameter thicker than that of the ultrafine fiber and the support treated with a sealer, and an adhesive layer laminated on one surface of the support, wherein at least the support is perforated, the perforation forms plural pores sequenced at least in one direction, the ratio (b/a) of interval a of the perforated part and interval b of the non-perforated part in one direction is 1 - 1.5, and the ratio (c/d) of the tensile strength c in the longitudinal direction and the tensile strength d in the width direction is 0.8 - 1.7.

According to the present invention, by laminating multiple layers of non-woven fabrics having different fiber diameters, seal-treating the laminate and using the laminate as a support, penetration of the adhesive to the backside of the support can be prevented when forming an adhesive layer and a decrease in the void ratio can be prevented. As a result, a medical adhesive tape or sheet having a superior texture, which can prevent stuffiness, skin irritation and the like can be provided. Moreover, since at least the support is perforated at a particular ratio, the tape or sheet can be easily cut with hands without using a cutting tool such as scissors and the like, thus providing a good cross-section.

Accordingly, the medical adhesive tape or sheet of the present invention is preferable for medical use and as hygiene materials such as an adhesive plaster, a covering material to cover a wound, a covering material used after surgical operation, a covering material for a needle puncture part of a catheter, a covering material such as gauze and the like, and the like. In addition, the medical adhesive tape or sheet can be preferably used for formation of a medical product in combination with other support etc., such as immobilization tape, instrument maintenance tape and the like. Furthermore, it is also useful as a tape for body shape reinforcement, amendment and the like in osteopathy and the like, or a taping tape for sports and the like.
Fig. 1(a) is a plane view showing one embodiment of the medical adhesive tape or sheet of the present invention, and Fig. 1(b) is a sectional view along line I-I of the adhesive tape.
Figs. 2(a), (b) and (c) are sectional views showing other embodiments of the medical adhesive tape or sheet of the present invention.
Figs. 3(a) and (b) are sectional views showing still other embodiments of the medical adhesive tape or sheet of the present invention.

1: support, 2: adhesive layer, 3: first non-woven fabric layer, 4: second non-woven fabric layer, 5: surface opposite to the surface of the support where the adhesive layer is formed, 6: pore, 10: medical adhesive tape or sheet, A: longitudinal direction, B: width direction, C: tip of pore, M: row interval, a: interval of perforation, b: interval of non-perforated part, α, β: angles.

The present invention is explained in detail in the following by referring to preferable embodiments thereof. In the explanation of the drawings, the same element is accorded with the same symbol and duplicate explanations are omitted. For the convenience of showing, the size ratio of the drawings is not necessarily the same as that in the explanation.

### (First embodiment)

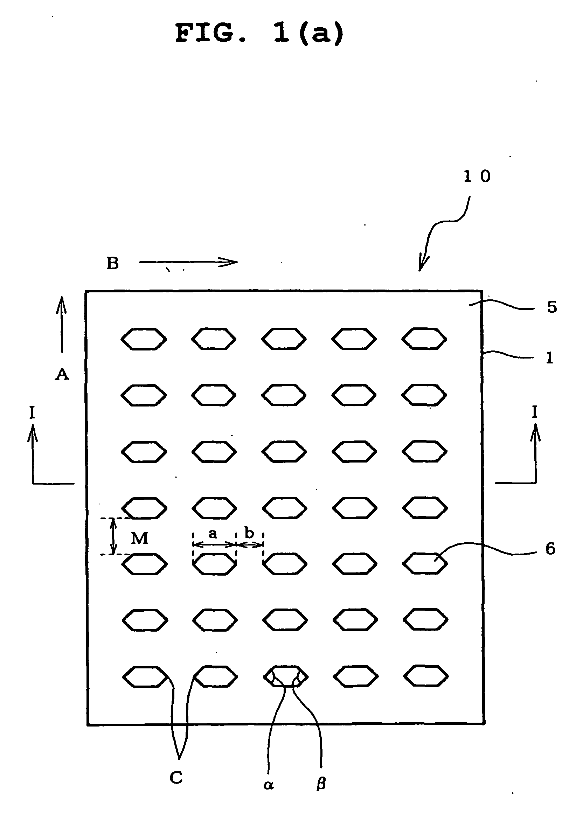

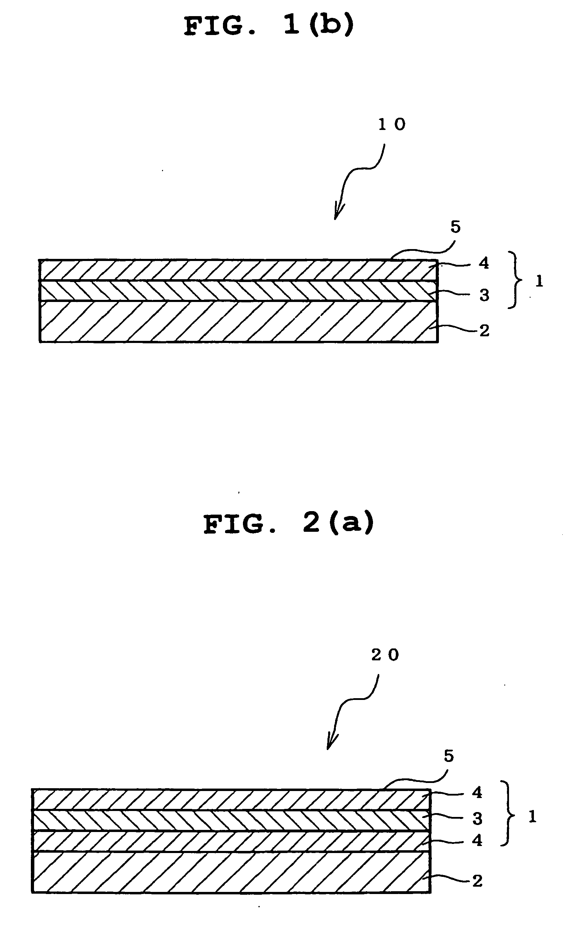

Fig. 1(a) is a plane view showing one embodiment of the medical adhesive tape or sheet of the present invention (hereinafter sometimes to be simply referred to as "an adhesive tape"), and Fig. 1(b) is a sectional view along line I-I of the adhesive tape. An adhesive tape 10 of this embodiment comprises a support 1, and an adhesive layer 2 laminated on one surface of the support 1. The support 1 is a multi-layer laminate of non-woven fabrics having different fiber diameters. In this embodiment, the laminate has a two-layer structure of a first non-woven fabric layer 3 and a second non-woven fabric layer 4, wherein the second non-woven fabric layer 4 is formed on a surface 5 opposite to the surface of the support 1 where the adhesive layer is formed. In the adhesive tape 10, at least the support 1 is perforated, forming plural pores 6.

The first non-woven fabric layer and the second non-woven fabric layer are not particularly limited as long as they are constituted with non-woven fabrics having different fiber diameters. The first non-woven fabric layer is preferably constituted with a non-woven fabric made of ultrafine fibers, and the second non-woven fabric layer is preferably constituted with a non-woven fabric made of fibers having a fiber diameter greater than that of the above-mentioned ultrafine fibers.

In the present specification, the ultrafine fiber means a fiber having a fiber diameter of not more than 5 µm (preferably 3 µm, more preferably not more than 2.5 µm, still more preferably not more than 2 µm), and the fiber length is not particularly limited. When the fiber diameter grows, the filling rate of the first non-woven fabric layer decreases and the amount used of the sealer increases. Consequently, the texture becomes poor and an adhesive layer cannot be formed easily by directly coating the support with an adhesive. Moreover, a comparatively large amount of a sealer is required for a sufficient seal-treatment, and an adhesive tape cannot be provided at a low cost. When the fiber diameter is too thin, the production efficiency generally decreases, and an economical production of a support having the above-mentioned constitution becomes difficult. Thus, a fiber having a fiber diameter of not less than 0.5 µm is desirably used. In the following, the first non-woven fabric layer is sometimes referred to as an "ultrafine fiber layer".

The fiber constituting the second non-woven fabric layer is not particularly limited as long as it has a fiber diameter greater than that of the above-mentioned ultrafine fiber. A fiber having a fiber diameter of generally above 5 µm and not more than 50 µm, preferably 8 - 20 µm, is used. When the fiber diameter is not more than 5 µm, the fabric becomes too fine and hard, its texture becomes poor, and may cause physical irritation to the skin. On the other hand, when it exceeds 50 µm, the surface becomes very uneven and the texture tends to become poor. The fiber length is not particularly limited. In the following, the second non-woven fabric layer is sometimes referred to as a "non-ultrafine fiber layer".

The material constituting the ultrafine fiber layer and the non-ultrafine fiber layer may be a synthetic fiber, other fibers, for example, natural fiber and the like and is not particularly limited. Specifically, natural fibers such as cotton, hemp, wool and the like, regenerated fibers such as rayon, cupra and the like, semi-synthetic fibers such as acetate, promix and the like, synthetic fibers such as nylon, polyester, acrylic, vinylon, polyvinyl chloride, vinylidene, polyolefin, polyurethane, polychlal, fluorocarbon, novoloid and the like, inorganic fibers such as glass fiber, carbon fiber, alumina fiber, silicon carbide fiber, slag fiber, metal fiber and the like, lumber, pulp and the like can be used. Of these, pulp fiber, polyester fiber and the like are preferable from the aspects of easy availability, stability, high melting point, hygroscopicity and the like. The ultrafine fiber layer and the non-ultrafine fiber layer may be constituted with the same material or different materials.

In addition, the production method of a non-woven fabric is not particularly limited, and a production method generally employed for the production of a non-woven fabric, such as dry method, wet method, spun bond method, melt blow method, flash spinning method, "toukaisen" film stretch method and the like can be employed. Specifically, the dry method includes adhesive type immersion method, print method, spray method, powder method, thermal bond method, and further, mechanical bond type felt method, stitch method, needle punch method, hydroentanglement type spun lace method, spinning type spun bond method, network structure method, melt blow method, film method and the like. The wet method includes hydroentanglement type spun lace method, spinning type spun bond method, flash method, thermal adhesion fiber method of paper making method, heat pressure method, adhesive method and the like.

Particularly, the ultrafine fiber layer is preferably a non-woven fabric produced by a melt blow method, and the non-ultrafine fiber layer is preferably a non-woven fabric produced by a spun bond method. As a result, penetration of an adhesive when forming an adhesive layer by directly applying the adhesive to a support can be prevented. Moreover, good texture is not impaired even by a treatment with a sealer, and stuffiness, skin irritation and the like can also be suppressed.

The support of this embodiment has a two-layer structure wherein a non-ultrafine fiber layer is laminated on an ultrafine fiber layer. The method of adhering the non-woven fabrics is not particularly limited, and a conventional method such as thermal adhesion, adhesion with an adhesive and the like can be employed.

While the ultrafine fiber layer and the non-ultrafine fiber layer do not need to have the same weight and fiber length, the weight of the whole non-woven fabric is preferably 10 - 80 g/m², and more preferably 12 - 60 g/m² in consideration of the texture and mechanical strength.

The thickness of the ultrafine fiber layer is preferably not more than 50 µm, more preferably not more than 30 µm. When the thickness exceeds 50 µm, the whole support becomes hard on seal-treatment, and the texture tends to be poor. For sufficient seal-treatment, the thickness of the ultrafine fiber layer is desirably not less than 5 µm. On the other hand, the thickness of the non-ultrafine fiber layer is preferably not more than 150 µm, more preferably not more than 100 µm. When the thickness exceeds 150 µm, the handling tends to become difficult during preparation of a support or an adhesive tape, and when it is less than 20 µm, the handling as well as texture tend to become poor. Therefore, the thickness of the non-ultrafine fiber layer is desirably not less than 20 µm.

The support is treated with a sealer as mentioned above. The sealer is not particularly limited as long as it causes less chemical irritation to the skin, can realize flexibility without causing a foreign sensation to the skin upon contact, and has moisture permeability. As such a sealer, for example, acrylic resin, vinylacrylic resin, acetate-rayon, polyvinyl acetate and the like can be used. Furthermore, latex bonded to acrylic resin, styrene-butadiene rubber, vinyl acetate-ethylene, vinyl acetate-acrylate, polyvinyl chloride, polyvinyl alcohol, polyurethane, vinyl acetate, acrylic-vinyl acetate and the like can be used. As water-based sealers including those similar to the above examples, acrylic latex binder, styrene-butadiene rubber latex, acrylic-vinyl acetate copolymer latex and the like can be used. The sealer may be of a solvent type, but an emulsion type is preferably used from the aspects of environmental protection.

The method of the sealer treatment includes, for example, a method comprising applying a water-based sealer to a non-woven fabric using a wound rod, a reverse roll, an air knife, a spray and the like. The amount of the sealer to be used is preferably determined as appropriate according to the desired properties, such as mechanical strength (drying temperature, wet strength) and tear property. It is generally 3 - 50 g/m², preferably 5 - 35 g/m². When it is less than 3 g/m², the seal-treatment tends to become insufficient, and an adhesive easily penetrates the support during direct application of the adhesive to the support to form an adhesive layer, where penetration to the backside of the support may occur. When it exceeds 50 g/m², the whole support becomes hard and the texture tends to be poor. When a sealer is applied to a support, the sealer is mainly present in the ultrafine fiber layer where the fibers are fine. Thus, the texture and breathability of the non-ultrafine fiber layer are not impaired. Accordingly, stuffiness, itchiness and the like can be suppressed while maintaining a superior texture.

The surface opposite to the surface of the support where the adhesive layer is formed (outermost layer of the support side) may be peel-treated. In this case, even when the adhesive tape is wound in a roll and preserved, the tape can be unwound smoothly. For a peel treatment, a peel treating agent is desirably applied to the support coated with a sealer. As the peel treating agent, silicone type, fluorine type, long chain alkyl type, wax and the like can be used. The amount of the peel treating agent to be used can be appropriately determined in consideration of the kind and the like of the adhesive.

As the adhesive with which to form an adhesive layer, one sufficiently flexible to follow the skin, less irritant to the skin (chemical stimulation, physical stimulation), and having moisture permeability is preferably used and, for example, an adhesive generally employed for a medical adhesive sheet can be used. As such an adhesive, for example, synthetic rubber adhesive comprising a styrene-isobutylene-styrene copolymer as a main component, polyurethane adhesive, polysiloxane adhesive, natural rubber adhesive, polyether adhesive, acrylic adhesive and the like can be mentioned, which may be used alone or two or more kinds thereof may be used in a mixture. Of these, acrylic adhesive is preferably used, and an acrylic adhesive containing a (meth)acrylic acid ester having an aliphatic hydrocarbon group such as butyl group, 2-ethylhexyl group, isononyl group, isooctyl group and the like as a monomer unit is preferable. From the aspect of improved cohesiveness and adhesiveness, an acrylic adhesive further containing (meth)acrylic acid as a monomer unit is more preferable. The acrylic polymer can be obtained by a known polymerization method such as solution polymerization, emulsion polymerization, suspension polymerization and the like. While solvent adhesives can also be used, emulsion adhesives are preferably used from the aspect of environmental protection.

The adhesive can contain various additives as necessary, such as plasticizers represented by polyvalent alcohols such as glycerol, polyethylene glycol, polypropylene glycol and the like, aqueous or water-absorbable resins such as polyacrylic acid, crosslinked polyacrylic acid, polyvinylpyrrolidone and the like, rosin, terpene and petroleum tackifiers, softener, filler, pigment, thickener and the like.

The thickness of the adhesive layer is preferably 10 - 100 µm, more preferably 20 - 70 µm. When the thickness of the adhesive layer is less than 10 µm, sufficient adhesiveness to the skin may not be afforded during application. When the thickness exceeds 100 µm, the water vapor permeability of the level requested of a medical adhesive sheet may not be achieved sufficiently. As a result, perspiration resistance is difficult to confer thereon, and skin irritation may be developed when the sheet is adhered for a long time.

The adhesive layer may be formed on the entire or partial surface of the support. When an adhesive layer is partially formed on a support, the adhesive layer may have a shape of a dot, streak and the like. In the case of a streak, a linear or a wavy line having a given width can be employed. The adhesive layer may be a laminate of two or more adhesive layers having different adhesive force. In this case, one adhesive layer may be formed on the entire surface of the support and the other adhesive layer may be partially formed thereon. The adhesive layer can be formed using an adhesive by solution coating, emulsion coating, or hot melt coating.

The adhesive layer may be directly or indirectly formed on one surface of a support. As used herein, "indirect" does not mean a direct contact of an adhesive layer with a support, but includes, for example, to improve the anchor property between an adhesive layer and a support, coating the surface of the support with a primer, and laminating the adhesive layer and the support via a layer formed by coating the primer.

The adhesive tape of this embodiment is perforated as mentioned above to form plural pores penetrating at least the support, wherein the pores may penetrate not only the support but also the adhesive layer. The perforation treatment is desirably applied to both the support and adhesive layer so that the tape can be easily cut with hands to form a good cross-section. While all perforated pores are desirably through-holes, they may not be completely penetrated but partially blocked as long as they do not exert an adverse influence on the hand-tearability. Being partially blocked means, after a perforation treatment of a support and an adhesive layer, only the support is penetrated, a part of the pore opening is blocked and the like.

The shape of the pore is not particularly limited and, for example, linear, circle, ellipse, polygon such as trapezoid, rectangle, hexagon, octagon and the like, flat polygon such as flat hexagon, flat octagon and the like, or deformed shape thereof can be mentioned. As the deformed shape, for example, a polygon wherein at least one side constituting itself is not a straight line but a curve or a wavy line can be mentioned. Of these, flat polygon, flat deformed polygon is preferable, and flat hexagon, flat deformed hexagon are more preferable.

The pores are set to form a row in at least one direction, and preferably formed in the longitudinal direction and width direction of an adhesive tape at given intervals. Moreover, the tips of the adjacent (opposing) pore are more preferably sequenced linearly in one direction. As a result, an adhesive sheet can be cut in the tip sequence direction. As used herein, the pore tip refers to, for example, the terminal portion thereof when the pore is linear, a convex of arc when the pore is circular, and a corner when the pore is polygon or flat polygon. The longitudinal direction of an adhesive tape refers to the direction of flow during production of a support (adhesive tape), and the width direction refers to the direction perpendicular to the longitudinal direction.

The size of the pore is not consistent due to the size of an adhesive tape. The area (opening area) is preferably 0.1 - 2.0 mm², more preferably 0.1 - 0.45 mm². When it is less than 0.1 mm², breathability and hand-tearability tend to be insufficient. When it exceeds 2.0 mm², the strength may decrease or sufficient adhesiveness may not be achieved with ease.

The constitution of the pore is specifically explained based on Fig. 1(a). In Fig. 1(a), flat hexagonal pores are sequenced in the longitudinal direction A and the width direction B at a given interval. The tips C of flat hexagon are sequenced linearly along the width direction, and the angles (α, β formed by two sides forming the tip C is a sharp angle of less than 90°. In the flat hexagon, at least one side may be constituted with a curve or a wavy line and, for example, the two sides forming the tip may be concave curves or wavy lines, or two sides parallel in the width direction may be concave curves, convex curves or wavy lines.

The ratio (b/a) of interval a of the perforated part and interval b of the non-perforated part in one direction is 1 - 1.5, preferably 1 - 1.3. When the ratio is less than 1, the hand-tearability becomes superior but the strength of the support becomes too low, thus permitting easy breakage during operation. When the ratio exceeds 1.5, the hand-tearability becomes insufficient.

When plural rows of the pores are present, the row interval is preferably 1 - 10 mm, more preferably 3 - 7 mm, so that the adhesive tape can be cut in a desired length. As used herein, the "row interval" means the interval between adjacent pores, as shown in Fig. 1(a), which refers to the interval M of the non-perforated part between the rows. When the row interval is less than 1 mm, the cross-section moves to the adjacent row during cutting since the row interval is too short, which makes it difficult to cut in the desired direction. When the tape is cut with hands, moreover, plural rows of pores are present on the fingers of both hands, which makes it difficult to cut at a desired position. When it exceeds 10 mm, the row interval is too wide and a pore may not be present where the tape is desired to be cut, thus degrading the operability.

The ratio (c/d) of the tensile strength c in the longitudinal direction to the tensile strength d in the width direction of an adhesive tape is 0.8 - 1.7. When the ratio is less than 0.8, the hand-tearability becomes superior but the operability is degraded because the tape becomes easily cut. When it exceeds 1.7, the hand-tearability becomes insufficient.

The non-perforated part between the pores sequenced in one direction may have a partially cut section. Once such cut section is formed, an adhesive tape can be certainly cut easily with hands. The shape, size, position and the like of the cut section is desirably determined in consideration of the balance of the strength of the adhesive sheet as a whole. The cut section may be set during a support formation process or after forming the support.

The pores are formed, for example, by die roll cutting with a blade, cutting by laser irradiation, perforation by a metal roll and the like. For perforation with a metal roll, for example, a metal roll having protrusions having a pore shape and a pressurization roll (metal roll with smooth surface) are disposed to face each other, and, for example, an adhesive sheet is passed between the rolls to form the pores. In this case, protrusions of the metal roll that form pores are disposed, for example, at a given interval, and the tip of the protrusions may be sharp or flat. As the pressurization roll, a stainless roll with a smooth surface, a rubber roll with a smooth surface which has a silicone rubber coated on the core metal and the like can be used.

The metal roll is preferably heated to about 150°C - 400°C, and the pressurization roll is preferably heated to about 50°C - 150°C.

The pressure applied between the metal roll and the pressurization roll, and the rate of passage (flowing rate) of an adhesive sheet between rolls are preferably determined in consideration of the shape of the protrusion of the metal roll, temperature of the protrusion, thickness of the adhesive sheet and the like, so that suitable pores are formed on the adhesive sheet. In general, the pressure between rolls is preferably about 10 - 200 kg/cm in a linear pressure and the flowing rate of an adhesive sheet is preferably about 1 - 50 m/min.

The adhesive tape of this embodiment may have a release sheet on an adhesive layer to protect the adhesive layer. As the release sheet, one wherein the contact side with an adhesive layer is peel-treated with silicone and the like can be used.

The form of the adhesive tape may be a flat plane (sheet) or a roll. An adhesive tape in a roll is preferable for preservation, transportation and the like and can be produced, for example, by forming an adhesive layer on an ultrafine fiber layer of a support, superimposing the adhesive layer on the surface of a non-ultrafine fiber layer of the support and winding the laminate in a roll. In this case, the back face of the support is desirably peel-treated. The size of the adhesive tape can be appropriately determined according to the object of use.

According to this embodiment, by laminating two layers of non-woven fabrics having different fiber diameters, seal-treating the laminate and using the laminate as a support, and setting a non-ultrafine fiber layer on the surface opposite to the surface of the support where the adhesive layer is formed, penetration of the adhesive to the backside of the support can be prevented when forming an adhesive layer and a decrease in the void ratio can be effectively prevented. As a result, a medical adhesive tape or sheet having a superior texture without impairing the good feeling that the non-ultrafine fiber layer has, which can prevent stuffiness, skin irritation and the like can be provided at a low cost. Moreover, since at least the support is perforated at a particular ratio, the tape or sheet can be easily cut in any length with hands without using a cutting tool such as scissors and the like, and the cross-section thereof is not uneven, thus providing a good cross-section.

Accordingly, the adhesive tape of this embodiment is preferable for medical use and as hygiene materials such as an adhesive plaster, a covering material to cover a wound, a covering material used after surgical operation, a covering material for a needle puncture part of a catheter, a covering material such as gauze and the like, and the like. In addition, the adhesive tape can be preferably used for formation of a medical product in combination with other support etc., such as immobilization tape, instrument maintenance tape and the like. Furthermore, it is also useful as a tape for body shape reinforcement, amendment and the like in osteopathy and the like, or a taping tape for sports and the like.

### (Second embodiment)

Fig. 2(a) is a sectional view showing another embodiment of the medical adhesive tape or sheet of the present invention. An adhesive tape 20 has a support 1 and an adhesive layer 2 laminated on one surface of the support 1. The support 1 has a three-layer structure containing a first non-woven fabric layer 3 and second non-woven fabric layers 4 laminated on both sides thereof, and the second non-woven fabric layer 4 is placed on a surface 5 opposite to the surface of the support 1 where the adhesive layer is formed. The two second non-woven fabric layers 4 constituting the support 1 may be the same or different. At least the support 1 of the adhesive tape 20 is perforated. As mentioned above, the adhesive tape of the present embodiment differs in the structure from the adhesive tape of the first embodiment having a two-layer structure in that the support has a three-layer structure. However, other constitutions, for example, the first and second non-woven fabric layers, an adhesive layer and perforation treatment, are as explained in the above-mentioned first embodiment.

Since the adhesive tape of this embodiment is constituted with a laminate of a three-layer structure, the support after a sealing treatment is certainly free from permeation of an adhesive and a decreased void ratio. Consequently, more superior usability can be realized and an adhesive tape particularly useful for medical application can be provided at a low cost.

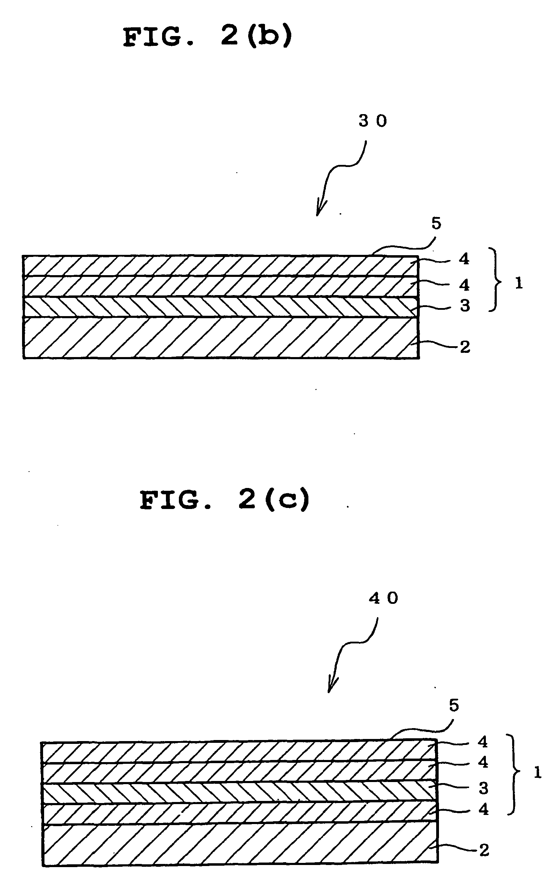

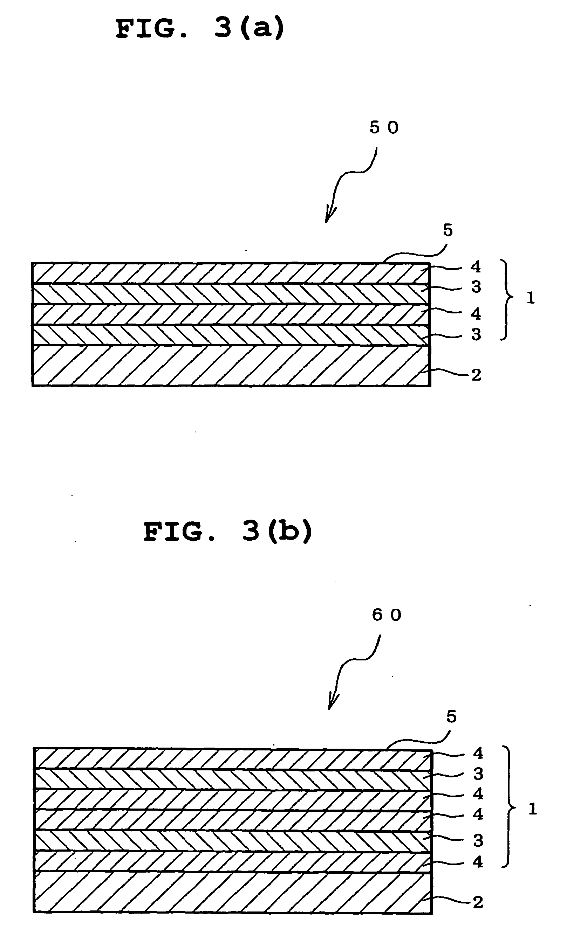

While the medical adhesive tape or sheet of the present invention has been explained in detail by referring to the embodiments thereof, the present invention is not limited to the above-mentioned embodiments. Various modifications may be made to the present invention without departing from the subject matter of the present invention. For example, as shown in Figs. 2(b) and (c), adhesive tapes 30 and 40 each having a support wherein one or more non-ultrafine fiber layers are further laminated on at least one surface of the support of the above-mentioned first or second embodiment may be provided. Alternatively, as shown in Figs. 3(a) and (b), adhesive tapes 50 and 60 each having a support wherein the support of the above-mentioned first or second embodiment as one unit is laminated in plurality may be provided.

### [Examples]

The present invention is explained in detail in the following by referring to Examples, which are not to be construed as limitative.

### (Preparation of emulsion adhesive)

A monomer mixture of 2-ethylhexylacrylate (95 parts by weight) and acrylic acid (5 parts by weight) was emulsified with sodium lauryl sulfate (emulsifier, 2 parts by weight), and emulsion polymerized using potassium persulfate (polymerization initiator, 0.2 part by weight) according to a conventional method to give an emulsion type acrylic adhesive with a solid concentration of 50 wt%. The weight average molecular weight of the solvent-soluble acrylic polymer in the obtained adhesive was 800,000, and the gel component (crosslinked) was 46 wt%.

### (Example 1)

To the both sides of a non-woven fabric (made from polyester, thickness 25 µm, fiber diameter 1.8 µm) made of ultrafine fibers and prepared by a melt blow method was bonded a non-woven fabric (made from polyester, thickness 45 µm, fiber diameter 13.5 µm) made of non-ultrafine fibers and prepared by a spun bond method. Thereafter, a sealing treatment was performed by impregnating the fabric with a sealer (25 g/m²) and drying same at 120°C for 3 min to give a support having the non-ultrafine fiber layer on both sides of the ultrafine fiber layer. The weight of the whole non-woven fabric was 25 g/m², and an acrylic emulsion (trade name; VONCOAT AB782E, manufactured by DAINIPPON INK AND CHEMICALS, INCORPORATED) was used as the sealer. Then, to the seal-treated support was applied the emulsion adhesive obtained above so that the weight after drying would be 50 g/m², and the support was dried at 120°C for 3 min and aged at 60°C for 3 days to form an adhesive layer. Next, a diluted peel treating agent (trade name; ASAHIGUARD AG8025, manufactured by MEISEI CHEMICAL WORKS, LTD.) was applied to the surface opposite to the surface where the adhesive layer was formed, dried at 120°C for 3 min to form a peel treated layer, whereby an adhesive sheet was obtained.

Using a perforating roll with flattened hexagonal protrusions, pores were formed on the adhesive sheet. The protrusions on the perforating roll had a horizontal width of 0.98 mm, a longitudinal width of 0.61 mm, a distance between protrusions in the width direction of 0.31 mm and a distance between protrusions in the longitudinal direction of 5.0 mm. The specific operation is as follows.

Setting the temperature of the perforating roll to 300°C and the temperature of the metal flat plane roll having a smooth surface to 100°C, an adhesive tape was compression passed between the perforating roll and the metal flat plane roll under a perforation pressure of 33 kgf/cm and at a rate of 20 m/min. As a result, an adhesive sheet as shown in Fig. 1, having pores penetrating the support and the adhesive layer, was obtained.

### (Example 2)

To the both sides of a non-woven fabric (made from polyester, thickness 25 µm, fiber diameter 1.8 µm) prepared by a melt blow method was bonded a non-woven fabric (made from polyester, thickness 45 µm, fiber diameter 14.3 µm) prepared by a spun bond method. Thereafter, the fabric was impregnated with the same sealer (20 g/m²) as in Example 1 to give a seal-treated support. The weight of the whole non-woven fabric was 25 g/m². Then, an adhesive layer was formed on the seal-treated support in the same manner as in Example 1. Next, in the same manner as in Example 1, a peel-treated layer was formed on the surface opposite to the surface where the adhesive layer was formed to give an adhesive sheet. In the same manner as in Example 1 except that the temperature of the perforating roll was set to 295°C, an adhesive sheet having pores penetrating the support and the adhesive layer was obtained.

### (Example 3)

In the same manner as in Example 2 except that the temperature of the perforating roll was set to 300°C, an adhesive sheet having pores penetrating the support and the adhesive layer was obtained.

### (Example 4)

In the same manner as in Example 2 except that the temperature of the perforating roll was set to 290°C, an adhesive sheet having pores penetrating the support and the adhesive layer was obtained.

### (Example 5)

In the same manner as in Example 2 except that the temperature of the perforating roll was set to 270°C, an adhesive sheet having pores penetrating the support and the adhesive layer was obtained.

### (Comparative Example 1)

A non-woven fabric (made from polyester, thickness 120 µm, fiber diameter 13.5 µm) prepared by a spun bond method was impregnated with the same sealer as in Example 1 to give a support having a single layer. The weight of the whole non-woven fabric was 25 g/m². Then, an adhesive was applied to the seal-treated support in the same manner as in Example 1 in an attempt to form an adhesive layer. However, the adhesive sank into the support and penetrated through the back face of the support and a good adhesive sheet could not be obtained.

### (Comparative Example 2)

To the both sides of a non-woven fabric (made from polyester, thickness 25 µm, fiber diameter 1.8 µm) prepared by a melt blow method was bonded a non-woven fabric (made from polyester, thickness 45 µm, fiber diameter 14.3 µm) prepared by a spun bond method to give a support. The weight of the whole non-woven fabric was 25 g/m². Then, without performing a sealing treatment, an adhesive was applied to the obtained support in the same manner as in Example 1 in an attempt to form an adhesive layer. However, as in Comparative Example 1, the adhesive sank into the support and penetrated through the back face of the support, and a good adhesive sheet could not be obtained.

### (Comparative Example 3)

In the same manner as in Example 2 except that the temperature of the perforating roll was set to 250°C, an adhesive sheet was prepared.

### (Comparative Example 4)

In the same manner as in Example 1 except that the amount of the sealer applied was set to 20 g/m² and the temperature of the perforating roll was set to 330°C, an adhesive sheet having pores penetrating the support and the adhesive layer was obtained.

### (Evaluation test)

### (tensile strength)

The tensile strength was measured using an autograph (type AG-IS, Shimadzu Corporation), for the adhesive sheets obtained in Examples 1 - 5 and Comparative Examples 3 and 4. The tensile strength was obtained by setting a distance between chucks to 100 mm and measuring the stress up to the breakage of the test piece (12 mm width) by pulling the piece at a tensile rate of 300 mm/min. This measurement operation was repeated 3 times each in the longitudinal direction and the width direction of the adhesive tape, and an average of the obtained values was determined.

### (ratio of interval of perforated part and interval of non-perforated part)

The intervals of the perforated part and the non-perforated part of the adhesive sheets obtained in Examples 1 - 5 and Comparative Examples 3 and 4 was measured using a microscope (type VQ-Z50, manufactured by KEYENCE CORPORATION). For the measurement, using an image of an adhesive sheet enlarged by 100 times, an average value of 15 measurement values in one direction at a 25 mm interval was obtained, and calculated by the following formula.
ratio of interval a of perforated part and interval b of non-perforated part = average of interval b of non-perforated portion/average value of interval a of perforated part (hand-tearability)

The adhesive sheets obtained in Examples 1 - 5 and Comparative Examples 3 and 4 were torn by fingers to give 25 mm long pieces in the width direction. The state at this time was evaluated according to the following evaluation criteria (n=5).
○: could be cut straight without deformation of the torn end
△: torn end was not deformed but the adjacent row was also cut
×: torn end was deformed, or the sheet could not be cut (cut surface)

The adhesive sheets obtained in Examples 1 - 5 and Comparative Examples 3 and 4 were torn in the width direction, and the cross-section was studied. The number of cross-section was counted and evaluated according to the following criteria (n=5).
○: single cross-section for 5 times and the sheet could be cut at a desired position
△: 1 or 2 cross-sections and the sheet could be cut at a desired position
×: two or more cross-sections for 5 times and the sheet could not be cut at a desired position

**Table 1**

| | first non-woven fabric layer (ultrafine fiber layer) | | second non-woven fabric layer (non-ultrafine fiber layer) | | amount (g/m²) of sealer applied | perforation treatment | perforation treatment (°C) | tensile strength | | | ratio of intervals of perforation part and non-perforation part | row interval (mm) | penetration of adhesive | hand-tearability | cut surface |
|---|---|---|---|---|---|---|---|---|---|---|---|---|---|---|---|
| | production method | fiber diameter (µm) | produc-tion method | fiber dia-meter (µm) | | | | longitudinal direction strength (N/12mm) | width direction strength (N/12mm) | strength ratio (longitudinal direction/ width direction) | | | | | |
| Ex. 1 | melt blow | 1.8 | spun bond | 13.5 | 25 | applied | 300 | 9.8 | 11.5 | 0.9 | 1.0 | 5 | ○ | ○ | ○ |
| Ex. 2 | melt blow | 1.8 | spun bond | 14.3 | 20 | applied | 295 | 14.7 | 9.2 | 1.8 | 1.1 | 5 | ○ | ○ | ○ |
| Ex. 3 | melt blow | 1.8 | spun bond | 14.3 | 20 | applied | 300 | 9.8 | 11.5 | 0.9 | 1.0 | 5 | ○ | ○ | ○ |
| Ex. 4 | melt blow | 1.8 | spun bond | 14.3 | 20 | applied | 290 | 15.4 | 10.8 | 1.4 | 1.1 | 5 | ○ | ○ | ○ |
| Ex. 5 | melt blow | 1.8 | spun bond | 14.3 | 20 | applied | 270 | 18.3 | 11.0 | 1.7 | 1.3 | 5 | ○ | △ | ○ |
| Comp. Ex. 1 | none | - | spun bond | 13.5 | 65 | - | - | - | - | - | - | - | × | - | - |
| Comp. Ex. 2 | melt blow | 1.8 | spun bond | 14.3 | 0 | - | - | - | - | - | - | - | × | - | - |
| Comp. Ex. 3 | melt blow | 1.8 | spun bond | 14.3 | 20 | applied | 250 | 23 | 10 | 2.3 | 1.6 | 5 | ○ | × | × |
| Comp. Ex. 4 | melt blow | 1.8 | spun bond | 13.5 | 20 | applied | 330 | 7.5 | 12.1 | 0.6 | 0.6 | 15 | ○ | △ | △ |

From each Example, it has been confirmed that an adhesive sheet having superior hand-tearability and a texture and free of penetration of an adhesive to the back side can be obtained by the use of a seal-treated, perforated support constituted with an ultrafine fiber and a non-ultrafine fiber. Particularly, it has also been confirmed that an adhesive tape more superior in the hand-tearability can be obtained when the ratio (b/a) of the interval a of the perforated part and the interval b of the non-perforated part is 1 - 1.5, the ratio (c/d) of the tensile strength c in the longitudinal direction and the tensile strength d in the width direction is 0.8 - 1.7, and the row interval is not more than 10 mm, as in Examples 1 and 2.

Moreover, from the Comparative Examples, it is clear that a support free of a specific laminate structure and a support with a specific laminate structure but free of a sealing treatment suffer from sinking of an adhesive, and cannot afford a desired adhesive sheet.

## Claims

1. A medical adhesive tape or sheet (10) comprising a support (1), which is a multi-layer laminate of non-woven fabrics having at least a first non-woven fabric layer (3) made of an ultrafine fiber and a second non-woven fabric layer (4) made of a fiber having a fiber diameter thicker than that of the ultrafine fiber and the support treated with a sealer, and an adhesive layer laminated on one surface of the support, wherein at least the support is perforated, the perforation forms plural pores (6) sequenced at least in one direction, the ratio (b/a) of interval a of the perforated part and interval b of the non-perforated part in one direction is 1 - 1.5, and the ratio (c/d) of the tensile strength c in the longitudinal direction and the tensile strength d in the width direction is 0.8 - 1.7.

2. The medical adhesive tape or sheet of claim 1, wherein the second non-woven fabric layer (4) is formed on the surface opposite to the surface of the support (1) where the adhesive layer is formed.

3. The medical adhesive tape or sheet of claim 1 or 2, wherein the support is a two-layer structure wherein the second non-woven fabric layer (4) is laminated on one surface of the first non-woven fabric layer (3), or a three-layer structure wherein the second non-woven fabric layer is laminated on each of the both surfaces of the first non-woven fabric layer.

4. The medical adhesive tape or sheet of any one of claims 1 to 3, wherein the first non-woven fabric layer (3) and the second non-woven fabric layer (4) are made from polyester.

5. The medical adhesive tape or sheet of any one of claims 1 to 4, wherein the first non-woven fabric layer (3) is prepared by a melt blow method.

6. The medical adhesive tape or sheet of any one of claims 1 to 5, wherein the second non-woven fabric layer (4) is prepared by a spun bond method.

7. The medical adhesive tape or sheet of claim 1, wherein the tips of the adjacent pores (6) are linearly sequenced in one direction.

8. The medical adhesive tape or sheet of claim 1 or 7, wherein the pores (6) are sequenced in plural rows arranged at a row interval of 1 - 10 mm.

9. The medical adhesive tape or sheet of any one of claims 1 to 8, wherein the treatment amount of the sealer is 3 - 50 g/m².

10. The medical adhesive tape or sheet of any one of claims 1 to 9, wherein the sealer is an acrylic resin.

11. The medical adhesive tape or sheet of any one of claims 1 to 10, wherein the adhesive layer is made from an acrylic adhesive.

12. The medical adhesive tape or sheet of any one of claims 1 to 11, wherein the surface opposite to the surface of the support (1) where the adhesive layer is formed is peel-treated.

13. The medical adhesive tape or sheet of any one of claims 1 to 12, which has a roll-like shape.

14. The medical adhesive tape or sheet of claim 1, wherein ultrafine fiber constituting the first non-woven fabric layer has a fiber diameter of not less than 0.5 µm and not more than 5 µm and the fiber constituting the second non-woven fabric layer has a fiber diameter of above 5 µm and not more than 50 µm.

## Patentansprüche

1. Medizinisches Klebeband oder -folie (10), umfassend einen Träger (1), bei dem es sich um ein mehrschichtiges Laminat aus Vliesstoffen handelt, das wenigstens eine erste Vliesstoffschicht (3), die aus einer ultrafeinen Faser besteht, und eine zweite Vliesstoffschicht (4), die aus einer Faser mit einem Faserdurchmesser, der dicker ist als bei der ultrafeinen Faser besteht, aufweist, wobei der Träger mit einem Dichtungsmittel behandelt ist und eine Kleberschicht auf eine Fläche des Trägers laminiert ist, wobei wenigstens der Träger perforiert ist, die Perforation mehrere Poren (6) bildet, die wenigstens in einer Richtung fortlaufend angeordnet sind, das Verhältnis (b/a) des Abstands a des perforierten Teils und des Abstands b des nichtperforierten Teils in einer Richtung 1 bis 1,5 beträgt und das Verhältnis (c/d) der Zugfestigkeit c in Längsrichtung und der Zugfestigkeit d in Richtung der Breite 0,8 bis 1,7 beträgt.

2. Medizinisches Klebeband oder -folie gemäß Anspruch 1, wobei die zweite Vliesstoffschicht (4) auf der Fläche gebildet ist, die der Fläche des Trägers (1), wo die Kleberschicht gebildet ist, gegenüberliegt.

3. Medizinisches Klebeband oder -folie gemäß Anspruch 1 oder 2, wobei der Träger eine zweischichtige Struktur ist, wobei die zweite Vliesstoffschicht (4) auf eine Fläche der ersten Vliesstoffschicht (3) laminiert ist, oder eine dreischichtige Struktur ist, wobei die zweite Vliesstoffschicht auf beide Flächen der ersten Vliesstoffschicht laminiert ist.

4. Medizinisches Klebeband oder -folie gemäß einem der Ansprüche 1 bis 3, wobei die erste Vliesstoffschicht (3) und die zweite Vliesstoffschicht (4) aus Polyester bestehen.

5. Medizinisches Klebeband oder -folie gemäß einem der Ansprüche 1 bis 4, wobei die erste Vliesstoffschicht (3) durch ein Schmelzblasverfahren hergestellt ist.

6. Medizinisches Klebeband oder -folie gemäß einem der Ansprüche 1 bis 5, wobei die zweite Vliesstoffschicht (4) durch ein Spinnvliesverfahren hergestellt ist.

7. Medizinisches Klebeband oder -folie gemäß Anspruch 1, wobei die Spitzen der benachbarten Poren (6) in einer Richtung linear fortlaufend angeordnet sind.

8. Medizinisches Klebeband oder -folie gemäß Anspruch 1 oder 7, wobei die Poren (6) in mehreren Reihen mit einem Reihenabstand von 1 bis 10 mm fortlaufend angeordnet sind.

9. Medizinisches Klebeband oder -folie gemäß einem der Ansprüche 1 bis 8, wobei die Behandlungsmenge des Dichtungsmittels 3 bis 50 g/m² beträgt.

10. Medizinisches Klebeband oder -folie gemäß einem der Ansprüche 1 bis 9, wobei das Dichtungsmittel ein Acrylharz ist.

11. Medizinisches Klebeband oder -folie gemäß einem der Ansprüche 1 bis 10, wobei die Kleberschicht aus einem Acrylkleber besteht.

12. Medizinisches Klebeband oder -folie gemäß einem der Ansprüche 1 bis 11, wobei die Fläche, die der Fläche des Trägers (1), wo die Kleberschicht gebildet ist, gegenüberliegt, mit einer Trennbeschichtung versehen ist.

13. Medizinisches Klebeband oder -folie gemäß einem der Ansprüche 1 bis 12, das bzw. die eine rollenartige Form hat.

14. Medizinisches Klebeband oder -folie gemäß Anspruch 1, wobei die ultrafeine Faser, die die erste Vliesstoffschicht bildet, einen Faserdurchmesser von nicht weniger als 0,5 µm und nicht mehr als 5 µm aufweist und die Faser, die die zweite Vliesstoffschicht bildet, einen Faserdurchmesser von über 5 µm und nicht mehr als 50 µm aufweist.

## Revendications

1. Bande ou feuille adhésive (10) à usage médical comprenant un support (1), qui est un stratifié multicouche de tissus non tissés comportant au moins une première couche de tissu non tissé (3) composée d'une fibre ultrafine et une seconde couche de tissu non tissé (4) composée d'une fibre ayant un diamètre de fibre plus épais que celui de la fibre ultrafine et du support traité à l'aide d'un produit d'isolation, et une couche adhésive stratifiée sur une surface du support, dans laquelle au moins le support est perforé, la perforation forme plusieurs pores (6) ordonnés au moins dans une direction, le rapport (b/a) d'intervalle a de la partie perforée sur intervalle b de la partie non perforée dans une direction est compris entre 1 et 1,5, et le rapport (c/d) de résistance à la traction c dans la direction longitudinale sur résistance à la tension d dans le sens de la largeur est compris entre 0,8 et 1,7.

2. Bande ou feuille adhésive à usage médical selon la revendication 1, dans laquelle la seconde couche de tissu non tissé (4) est formée sur la surface opposée à la surface du support (1) où est formée la couche adhésive.

3. Bande ou feuille adhésive à usage médical selon la revendication 1 ou 2, dans laquelle le support est une structure à deux couches, la seconde couche de tissu non tissé (4) étant stratifiée sur une surface de la première couche de tissu non tissé (3), ou une structure à trois couches, la seconde couche de tissu non tissé étant stratifiée sur chacune des deux surfaces de la première couche de tissu non tissé.

4. Bande ou feuille adhésive à usage médical selon l'une quelconque des revendications 1 à 3, dans laquelle la première couche de tissu non tissé (3) et la seconde couche de tissu non tissé (4) sont fabriquées à partir de polyester.

5. Bande ou feuille adhésive à usage médical selon l'une quelconque des revendications 1 à 4, dans laquelle la première couche de tissu non tissé (3) est préparée par un procédé de fusion-soufflage.

6. Bande ou feuille adhésive à usage médical selon l'une quelconque des revendications 1 à 5, dans laquelle la seconde couche de tissu non tissé (4) est préparée par un procédé de liaison-filage.

7. Bande ou feuille adhésive à usage médical selon la revendication 1, dans laquelle les pointes des pores adjacentes (6) sont ordonnées linéairement dans une direction.

8. Bande ou feuille adhésive à usage médical selon la revendication 1 ou 7, dans laquelle les pores (6) sont ordonnés sur plusieurs rangées agencées selon un intervalle de rangée de 1 à 10 mm.

9. Bande ou feuille adhésive à usage médical selon l'une quelconque des revendications 1 à 8, dans laquelle la quantité de traitement du produit d'étanchéité est comprise entre 3 et 50 g/m².

10. Bande ou feuille adhésive à usage médical selon l'une quelconque des revendications 1 à 9, dans laquelle le produit d'étanchéité est une résine acrylique.

11. Bande ou feuille adhésive à usage médical selon l'une quelconque des revendications 1 à 10, dans laquelle la couche adhésive est fabriquée à partir d'un adhésif acrylique.

12. Bande ou feuille adhésive à usage médical selon l'une quelconque des revendications 1 à 11, dans la surface opposée à la surface du support (1) où est formée la couche adhésive est traitée pour décollement.

13. Bande ou feuille adhésive à usage médical selon l'une quelconque des revendications 1 à 12, qui a la forme d'un rouleau.

14. Bande ou feuille adhésive à usage médical selon la revendication 1, dans laquelle la fibre ultrafine constituant la première couche de tissu non tissé a un diamètre de fibre de pas moins de 0,5 µm et de pas plus de 5 µm et la fibre constituant la seconde couche de tissu non tissé a un diamètre de fibre de plus de 5 µm et de pas plus de 50 µm.
